# EUROPEAN PATENT SPECIFICATION

(11) **EP 4 337 283 B1**
(45) Date of publication and mention of the grant of the patent: **09.12.2026**
(21) Application number: 22726968.5
(22) Date of filing: 12.05.2022
(51) Int. Cl.: A61M 5/142, F04B 9/04, A61M 5/168, A61M 5/172, A61M 5/145

(54) **DEVICES FOR DETERMINING FLUID DELIVERY PUMP INFORMATION**
VORRICHTUNGEN ZUR BESTIMMUNG VON INFORMATIONEN ÜBER FLÜSSIGKEITSFÖRDERPUMPEN
DISPOSITIFS POUR DÉTERMINER DES INFORMATIONS DE POMPE DE DISTRIBUTION DE FLUIDE

(30) Priority: 12.05.2021 US 202163187595 P
(43) Date of publication of application: 20.03.2024
(60) Divisional application: 26213237.6
(73) Proprietor: Insulet Corporation, Acton, MA 01720 (US)
(72) Inventor: CARDINALI, Steven, Tewksbury, Massachusetts 01876 (US); BISSON, Brian, Dracut, Massachusetts 01826 (US); BREINGAN, Kyle, Lowell, Massachusetts 01852 (US)
(74) Representative: Peterreins Schley
(86) International application number: PCT/US2022/029012
(87) International publication number: WO 2022/241128

(56) References cited:
- EP-B1- 1 874 390
- US-A- 4 277 226
- US-A1- 2016 367 754

## Description

### FIELD OF THE INVENTION

The present disclosure generally relates to a pump system for infusing a patient with a fluid, for example, a pump arranged within a wearable medicament delivery system, and, in particular, a pump system configured to determine pump cycle or sequencing information.

### BACKGROUND

Healthcare providers may prescribe patients wearable devices for delivering fluids, such as liquid medicaments, as part of a treatment regimen. Non-limiting examples of medicaments may include chemotherapy drugs, hormones (for instance, insulin), pain relief medications, and other types of liquid-based drugs. In general, wearable medicament delivery devices are relatively small form factors that may be adhered to the skin of the patient, with a reservoir to hold the medicament. The device may include a needle or cannula fluidically coupled to the reservoir and extending from the device and into the skin of the patient. A pump may operate to force the fluid from the reservoir, through a fluid path, and out through the needle and into the patient. A control system, with hardware and/or software elements, may be arranged within the device to manage medicament delivery and other device features. The control system may operate alone or in combination with an external computing device, such as a patient smartphone, healthcare provider computer, and/or the like. Minimizing the footprint of a wearable medicament delivery device makes the device less obtrusive to the patient and improves the overall user experience. Accordingly, the dimensions of operational devices, such as fluid pumps, are kept as small as possible.

Determining operational information for a wearable medicament delivery device and individual components is key to maintaining proper functioning and ensuring patient safety during use. However, smaller component sizes and footprint constraints make it more challenging to sense component status information. For example, the pump chamber volume is typically much smaller than the device's fluid reservoir volume and is therefore refilled periodically. A pump may use various mechanisms to initiate a refill process to refill the pump chamber with a fluid. Before this refill process occurs, the exit port to the patient must be closed and the entry port to the reservoir must be opened. With conventional pump systems that are dimensioned for a wearable medicament delivery device, it is challenging to efficiently and accurately determine when the fill process is going to occur with sufficient time so that the switch can happen, particularly if there is an error.

US 4 277 226 A relates to a volumetric infusion pump useful in intravenous feeding including a detector which provides an alarm when the supply bag or reservoir becomes empty or an occlusion occurs between the supply bag and the pump. The pump includes a first pumping chamber having a cylinder, a piston, a first flexible diaphragm and first and second valves. The piston is driven by a first cam through a first cam follower which is connected to the piston. The empty supply bag detector senses contact between the first cam and the first cam follower. During normal operation, when the first valve opens and the second valve closes the first pumping chamber fills due to the head pressure of fluid from the bag, and the cam follower remains in contact with the cam. When the supply bag becomes empty due to an improper setup or a system malfunction such as an occlusion occurs, head pressure in the fluid being supplied to the pumping chamber is lost, and the cam follower no longer can maintain contact with the cam during the fill portion of the pumping cycle. This results in an alarm signal which stops the pump.

EP 1 874 390 B1 relates to a fluid delivery device including a fluid driving mechanism for driving fluid out of a reservoir and an actuating mechanism for actuating the driving mechanism. The fluid delivery device may also include one or more sensors for monitoring operation of the fluid delivery device. The fluid delivery device may also include a chassis providing mechanical and/or electrical connections between components of the fluid delivery device.

US 2016/367754 A relates to a fluid infusion device including a pump having a rotor and a stator. A rotor cam element rises from a reference surface of the rotor. The stator includes a cam element having a stator cam surface. The cam elements axially displace the rotor as the rotor revolves. Inlet and outlet valves open and close as a function of angular and axial position of the rotor. A motor actuates the rotor to pump fluid to a body, via a subcutaneous conduit. First and second contact elements are located on the rotor. A sensing element on the stator cooperates with a detection circuit to detect when the sensing element makes contact with the first sensor contact element and the second sensor contact element. The detection circuit monitors a detection signal obtained from the sensing element to determine an operating condition of the fluid pump mechanism.

Therefore, there is a need for an improved pumping mechanism for a wearable medicament delivery device that can accurately and efficiently determine status information for device components, such as a fluid pump.

### BRIEF DESCRIPTION OF THE DRAWINGS

**FIG. 1** illustrates an exemplary operating environment in accordance with the present disclosure;
**FIG. 2** illustrates an exemplary wearable fluid delivery device in accordance with the present disclosure;
**FIGs. 3A-3B** illustrate an embodiment of a fluid delivery pump in accordance with the present disclosure;
**FIGs. 4A-4B** illustrate an embodiment of a fluid delivery pump in accordance with the present disclosure;
**FIGs. 5A-5D** illustrate an embodiment of a fluid delivery pump in accordance with the present disclosure;
**FIG. 6** illustrates an embodiment of a rotary element of a fluid delivery pump in accordance with the present disclosure; and
**FIGs. 7A-7B** illustrate an embodiment of a fluid delivery pump in accordance with the present disclosure.

The drawings are not necessarily to scale. The drawings are merely representations, not intended to portray specific parameters of the disclosure. The drawings are intended to depict example embodiments of the disclosure, and therefore should not be considered as limiting in scope. In the drawings, like numbering represents like elements.

### SUMMARY

The present invention is defined in the appended claims.

### DETAILED DESCRIPTION

The described technology generally relates to a wearable fluid delivery device for delivering a fluid to a patient. In some embodiments, the fluid may be or may include a medicament. The wearable fluid delivery device may include a reservoir for holding the fluid, a fluid path in fluid communication with the reservoir, a needle in fluid communication with the fluid path to deliver the fluid to the patient wearing the wearable fluid delivery device, and a fluid delivery pump configured to force the fluid from the reservoir, through the fluid delivery path, and into the patient via the needle.

In some embodiments, a fluid pump may include a pump location determination assembly or pump status assembly configured to determine a step, process, sequence, state, component location, component orientation, or other operational information of a fluid delivery pump. In various embodiments, the pump status assembly may operate to determine status or location information (for instance, where a rotating member is in a rotation cycle) for one or more components of the fluid pump. Accordingly, wearable fluid delivery device control components may use the status information to monitor device operations and/or perform functions based on the status information.

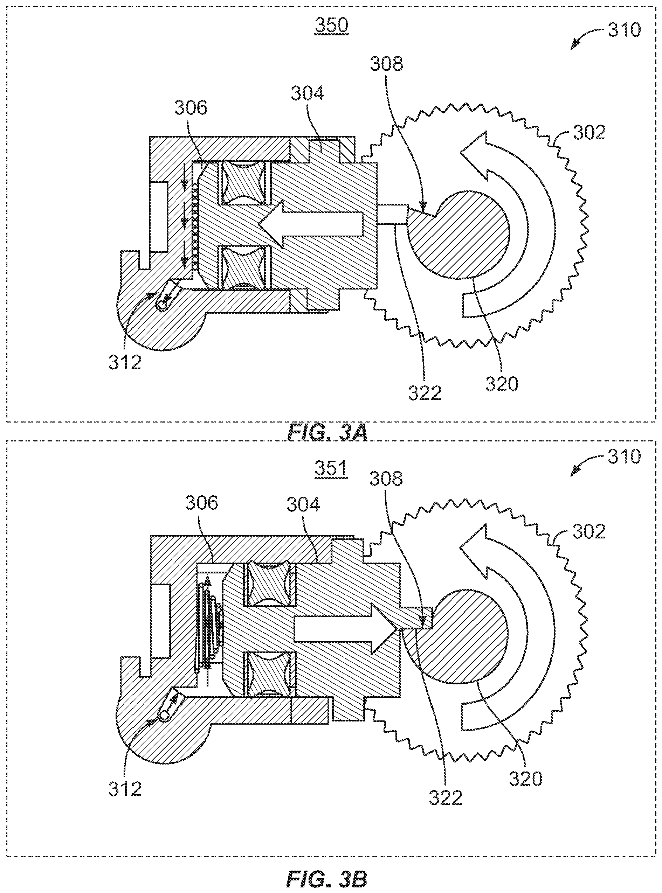

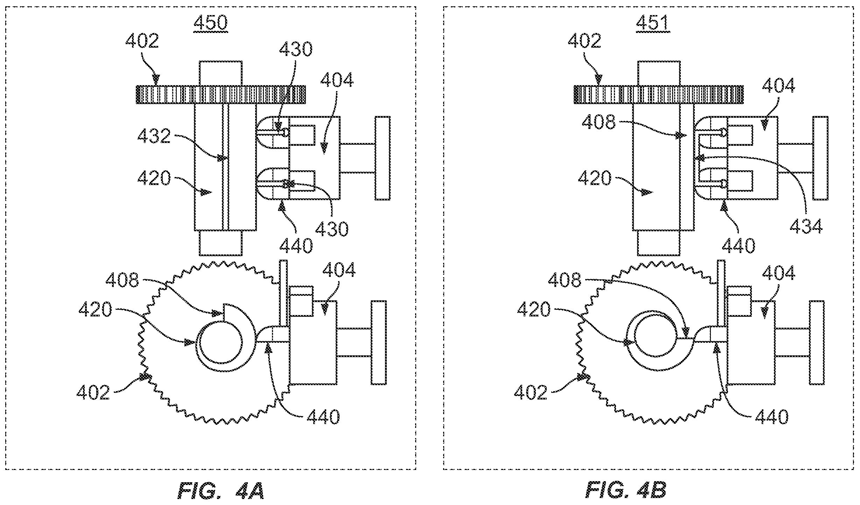

For example, in some embodiments, a fluid pump may be or may include a reciprocating pump (see, for example, FIGS. 3A-3B and 4A-4B). In various embodiments, the fluid pump may use a spiral shaped "snail cam" to turn rotary motion into linear motion, thereby allowing the wearable fluid delivery device to deliver small pulses (for instance, less than 0.5 microliters). The pump chamber volume is smaller than the device's reservoir volume and, therefore, is refilled periodically or cyclically. The refill mechanism may include a drop-off in the snail cam with a spring force that refills the pump chamber with the device fluid. Before the snail cam drop-off occurs, the exit port to the patient must be closed and the entry port to the reservoir must be opened. It must be determined when the snail cam drop-off is coming so that the switch can happen and so that fluid can flow in the proper direction (e.g., from the reservoir or to the patient). In conventional systems, an open loop system is used involving counting the number of ratchets or other mechanical steps of a ratchet, wheel, or other rotary member to recognize where the pump is in the pumping cycle. If an error occurs in the rotary or ratchet mechanism and a step is skipped or a step is not completed, then the count will be off, which may result in pulling fluid from the patient instead of the reservoir or other unwanted device behavior.

Accordingly, in some embodiments, a pump status assembly may include electronic sensors that provide the ability to recognize where one or more pump components, such as a piston, ratchet wheel, and/or other components are located in a pump cycle. For example, the pump status assembly may determine where a pump piston and/or ratchet wheel are with respect to the snail cam and, more importantly, with respect to a snail cam drop-off. Therefore, in some embodiments, a pump status assembly may provide a feedback system that will allow a fluid pump (or device control elements) to recognize when the snail cam drop-off is approaching and switch the pump from the patient to the reservoir accordingly.

In some embodiments, a pump status assembly may be configured to determine operational information associated with a fluid pump. In various embodiments, the pump status assembly may operate to determine a position, location, orientation, state, and/or other status of a pump element. The pump status assembly may include a first electrical element configured to engage a second electrical element to indicate the pump status. For example, the first electrical element may engage the second electrical element to form a closed circuit. The operational information may include a signal that a circuit has been closed and/or that a circuit has been opened (for instance, a binary 1/0 or on/off signal), and/or electrical information associated with a circuit (or lack thereof). Non-limiting electrical information may include, without limitation, resistance, voltage, amperage, inductance, capacitance, capacitor charge, and/or the like.

For example, a circuit may be formed (or alternatively, opened) responsive to engagement of a first pump element with a second pump element. The formation (or alternatively, breaking) of the circuit may be a signal of a pump event (such as switching from a fluid fill mode to a fluid delivery mode). In another example, a circuit may have different electrical properties based on engagement, position, or other status of pump components. For example, a resistance of a circuit may change as a first element travels with respect to a second element. A control element may determine status information (such as a location of the first element and/or second element) based on the change in resistance. The measured resistance may be compared to known resistance values that are indicative of a particular location of pumping elements or a pump status. As such, a threshold resistance may indicate a pump event or the location of a pump component.

The pump information or pump event may be used to control operational aspects of a fluid pump, such as changing fluid paths, activating pump elements, sending messages to a control device, error handling, and/or the like.

Although a snail cam and a piston are used in examples described in the present disclosure, embodiments are not so limited, as pump status assemblies may be used with various other types of pump components to determine a status of the component.

Other embodiments are contemplated in the present disclosure.

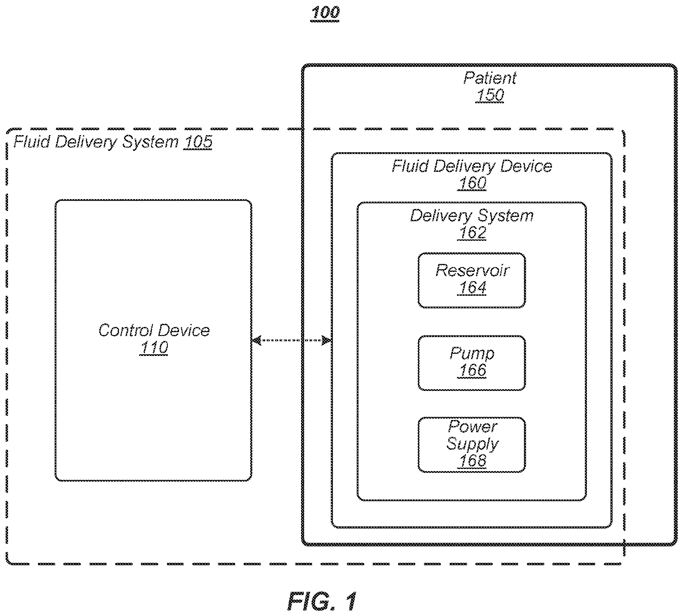

**FIG. 1** illustrates an example of an operating environment 100 that may be representative of some embodiments. As shown in FIG. 1, operating environment 100 may include a fluid delivery system 105. In various embodiments, fluid delivery system 105 may include a control or computing device 110 that, in some embodiments, may be communicatively coupled to a fluid delivery device 160; or may be physically integrated with fluid delivery device 160; or may be a combination of both: computing device 110 may represent (i) a remote control device that can control fluid delivery device 160 and (ii) a controller internal to fluid delivery device 160 that may control fluid delivery device 160 when not being controlled by the remote control device. Computing device 110 may comprise a processor and a memory and may be or may include one or more logic devices, including, without limitation, a server computer, a client computing device, a personal computer (PC), a workstation, a laptop, a notebook computer, a smart phone, a tablet computing device, a personal diabetes management (PDM) device, and/or the like. Embodiments are not limited in this context.

Fluid delivery device 160 may be or may include a wearable automatic fluid delivery device directly coupled to patient 150, for example, directly attached to the skin of the user via an adhesive and/or other attachment component. Fluid delivery device 160 may comprise one or more housings that house different elements of the fluid delivery device, for example, in the same housing or in different housings that connect together.

In some embodiments, fluid delivery device 160 may be or may include a medicament delivery device configured to deliver a liquid medicament, drug, therapeutic agent, or other medical fluid to a patient. Non-limiting examples of medicaments may include insulin, glucagon, glucagon like peptide (e.g., GLP-1), pramlintide, pain relief drugs, hormones, blood pressure medicines, morphine, methadone, chemotherapy drugs, proteins, antibodies, a combination of two or more of the foregoing, and/or the like.

In some embodiments, fluid delivery device 160 may be or may include an automatic insulin delivery (AID) device configured to deliver insulin (and/or other medication) to patient 150. For example, fluid delivery device 160 may be or may include a device the same or similar to an OmniPod^{®} device or system provided by Insulet Corporation of Acton, Massachusetts, United States, for example, as described in United States Patent Nos. 7,303,549; 7, 137,964; and/or 6,740,059. Although an AID device and insulin are used in examples in the present disclosure, embodiments are not so limited, as fluid delivery device 160 may be or may include a device capable of storing and delivering any fluid therapeutic agent, drug, medicine, hormone, protein, antibody, and/or the like, including those mentioned above.

Fluid delivery device 160 may include a delivery system 162 having a number of components to facilitate automated delivery of a fluid to patient 150, including, without limitation, a reservoir 164 for storing the fluid, a pump 166 for transferring the fluid from reservoir 164, through a fluid path or conduit, and into the body of patient 150, and/or a power supply 168. Fluid delivery device 160 may include at least one penetration element (not shown) configured to be inserted into the skin of the patient to operate as a conduit between reservoir 164 and patient 150. For example, penetration element may include a cannula and/or a needle. Embodiments are not limited in this context, for example, as delivery system 162 may include more or fewer components.

In some embodiments, computing device 110 may be a smart phone, PDM, or other mobile computing form factor in wired or wireless communication with fluid delivery device 160. For example, computing device 110 and fluid delivery device 160 may communicate via various wireless protocols, including, without limitation, Wi-Fi (i.e., IEEE 802.11), radio frequency (RF), Bluetooth^{™}, Zigbee^{™}, near field communication (NFC), Medical Implantable Communications Service (MICS), and/or the like. In another example, computing device 110 and fluid delivery device 160 may communicate via various wired protocols, including, without limitation, universal serial bus (USB), Lightning, serial, and/or the like. Although computing device 110 (and components thereof) and fluid delivery device 160 are depicted as separate devices, embodiments are not so limited. For example, in some embodiments, computing device 110 and fluid delivery device 160 may be a single device. In another example, some or all of the components of computing device 110 may be included in fluid delivery device 160. For example, fluid delivery device 160 may include processor circuitry, memory unit, and/or the like. In some embodiments, each of computing device 110 and fluid delivery device 160 may include a separate processor circuitry, memory unit, and/or the like capable of facilitating insulin/medicament infusion processes according to some embodiments, either individually or in operative combination. Embodiments are not limited in this context.

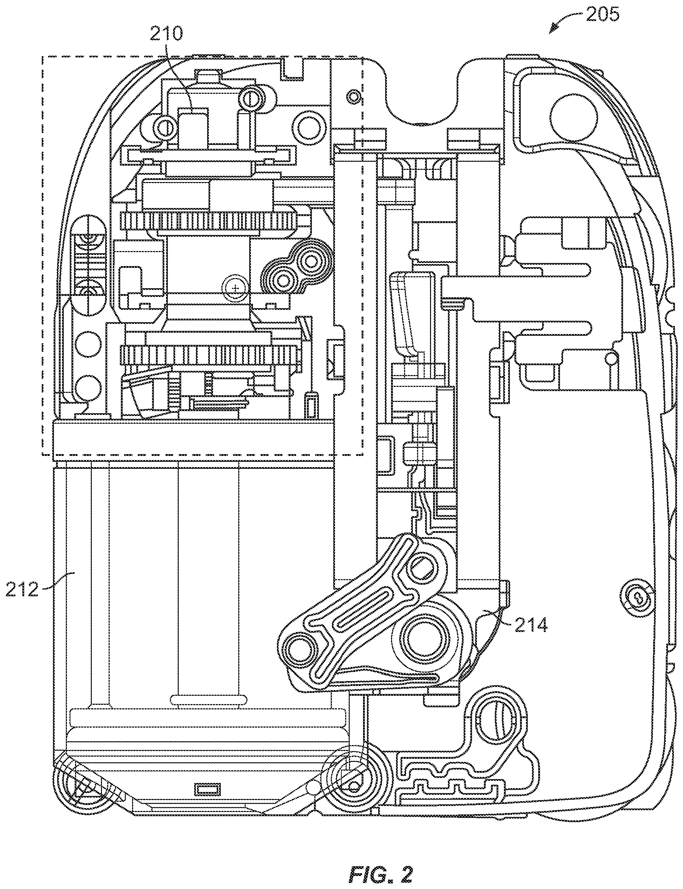

**FIG. 2** illustrates an exemplary wearable fluid delivery device in accordance with the present disclosure. In particular, FIG. 2 depicts a top-down view of a wearable fluid delivery device 205. As shown in FIG. 2, a wearable fluid delivery device 205 may include multiple systems to store and delivery a fluid to a patient. In some embodiments, wearable fluid delivery device 205 may include a pump 210. In various embodiments, pump 210 may be or may include a reciprocating pump (see, for example, FIGS. 3A-3B and 4A-4B). In exemplary embodiments, wearable fluid delivery device 205 may include a reservoir 212 for storing a fluid. Reservoir may be in fluid communication with pump 210 for delivering the fluid to patient via needle 214.

In various embodiments, pump 210 may be a multi-dose reciprocating pump. In some embodiments, pump 210 may be configured to deliver about 0.25 microliters per pulse. In exemplary embodiments, pump 210 may have a footprint of about 23 millimeters (mm) × 28 mm × 12 mm.

**FIGs. 3A-3B** illustrate an embodiment of a fluid delivery pump in accordance with the present disclosure. As shown in FIGs. 3A-3B, a fluid delivery pump 310 may include at least one ratchet or ratchet wheel 302 operably coupled to a snail cam 320. Rotation of ratchet 302 may cause corresponding rotation of snail cam 320. In some embodiments, rotation of ratchet 302 may be continuous over a specified duration. In other embodiments, rotation of ratchet 302 may be pulse-based, for example, rotating for an instructed number of pulses. As snail cam 320 rotates, a projection 322 of a piston 304 may ride along an outside surface of snail cam 320. Fluid delivery pump 310 may operate in one of two different modes, including a fluid delivery mode 350 (for example, infusing fluid to the patient) and a fluid fill mode 351 (filling a pump chamber 306 with fluid from a reservoir (not shown)). In fluid delivery mode 350, a fluid path (not shown) is open from chamber 306 to patient. In fluid fill mode 351, the fluid path is open from chamber 306 to the reservoir.

In fluid delivery mode 350, pump chamber 306 is at least partially full of fluid (for instance, from a previous fluid fill mode 350). Rotation of ratchet 302 may cause rotation of snail cam 320. Projection 322 of piston 304 may ride along an outside surface of snail cam 320, pushing piston 304 into chamber 306 and, therefore, expelling the fluid out of chamber 306 and through needle 312 to patient.

In fluid fill mode 351, when piston 304 engages a drop off 308 of snail cam 320, piston 304 moves in a direction away from chamber 306 (toward the right in FIGs. 3A-3B), causing negative pressure such that fluid flows from the reservoir into chamber 306.

The switch of the fluid delivery path from a fluid delivery path (for instance, a chamber-to-patient path) to a fluid fill path (for instance, a reservoir-to-chamber path) must occur when piston 304 engages drop off 308, otherwise the negative pressure resulting from movement of piston 304 away from chamber may negatively affect the patient, for example, drawing fluid from the patient rather than from the reservoir. Accordingly, some embodiments may provide a pump status assembly to indicate to pump control components to switch from a fluid delivery path to a fluid fill path at or before drop off 308 is encountered by piston 304.

**FIGs. 4A-4B** illustrate an embodiment of a fluid delivery pump in accordance with the present disclosure. As shown in FIGs. 4A-4B, a fluid pump may include a rotary element, such as a ratchet 402 operably coupled to a snail cam 420 having a drop off 408. In some embodiments, a fluid pump may include a pump status assembly, for example, formed from electronic elements 432 and 440. In some embodiments, pump status assembly may include other electrical components, such as capacitors, signal transmission lines, and/or the like to allow pump status assembly to operate according to some embodiments.

For example, snail cam 420 may have an electronic element 432, such as a conductive strip, arranged on a surface thereof. A piston 404, or other pump component, may engage snail cam 420 during rotation of ratchet 402. In various embodiments, piston 404 may include at least one electrical element, such as a conductive post or other projection 440. When post 440 engages conductive strip 432, a completed circuit 434 is formed. A signal from completed circuit 434 may be received by one or more control components of a fluid delivery device. When post 440 is not engaged with or touching conductive strip 432, pump status assembly has an open circuit. In some embodiments, a signal that the circuit is open may be provided to control components of a fluid delivery device. Accordingly, in some embodiments, pump status assembly may operate as a "snail cam drop sensor" configured to detect when a portion of the pump, such as piston 404, is about to or has engaged drop off 408.

In some embodiments, conductive strip 432 may be arranged at a position at or near drop off 408 (for instance, at or near the end of the snail cam "ramp"). In this manner, closed circuit 434 may be formed and may cause a signal or pulse to be sent to indicate that piston 404 is about to reach drop off 408, and the fluid path should be switched from a fluid delivery path to a fluid fill path. As piston 404 moves off of drop off 408, the circuit may be opened to signal a switch of the fluid path associated with the fluid pump.

Although electrical element 432 is depicted as a conductive strip and electrical element 440 is depicted as a pair of conductive projections in FIGs. 4A-4B, embodiments are not so limited, as electrical elements 432 and 440 may have various shapes, sizes, and/or structures and operate according to some embodiments. For example, electrical elements 432 and/or 440 may include one or more strips, wires, protrusions, ridges, slots, flanges, and/or the like. In some embodiments, electrical elements 432 and 440 may be formed of various materials capable of forming a circuit, including, without limitation, a metal, a conductive polymer, copper, silver, printed circuit boards (PCBs), and/or the like.

In various embodiments, electrical element 432 may be placed on or substantially on drop off 408 to signal when piston 404 has engaged drop off 408. In some embodiments, electrical element 432 may be placed a specified distance from drop off 408 to provide an indication that piston 404 is about to engage drop off 408. For example, the specified distance may be based on a set number of pulses (for instance, to predict piston 404 engagement with drop off 408 a set number of pulses (for example, 1-3 pulses) before the drop), a distance (for instance, a set number of millimeters before piston 404 engages drop off 408), percentage of snail cam 420 outer diameter, a time (for instance, a set number of milliseconds before piston engages drop off), and/or the like. Accordingly, a signal may be generated to indicate that piston 404 is a set a distance away from drop off 408. In some embodiments, electrical element 432 may include a plurality of elements configured to engage electrical element 440 at various locations along the outer surface of snail cam 420 as it rotates during pump operation.

As indicated in FIGs. 4A-4B, applying a conductive strip at the end of the snail cam ramp may allow a circuit to close when the snail cam is reaching the drop-off position. The open circuit contact points may be on the piston shaft pushing up against the snail cam. The closing of the circuit may directly activate the patient-to-reservoir switch that is required to refill the pump chamber. In other words, the pump chamber 306 may be fluidly connected to reservoir 212 when the snail cam reaches the drop-off position and as projection 322 or projection(s) 440 move down the drop-off (thereby filling the pump chamber 306); and the pump chamber 306 may be fluidly connected with the patient as the snail cam rotates up until projection 322/440 reaches the drop-off position again (thereby dispensing the fluid in pump chamber 306).

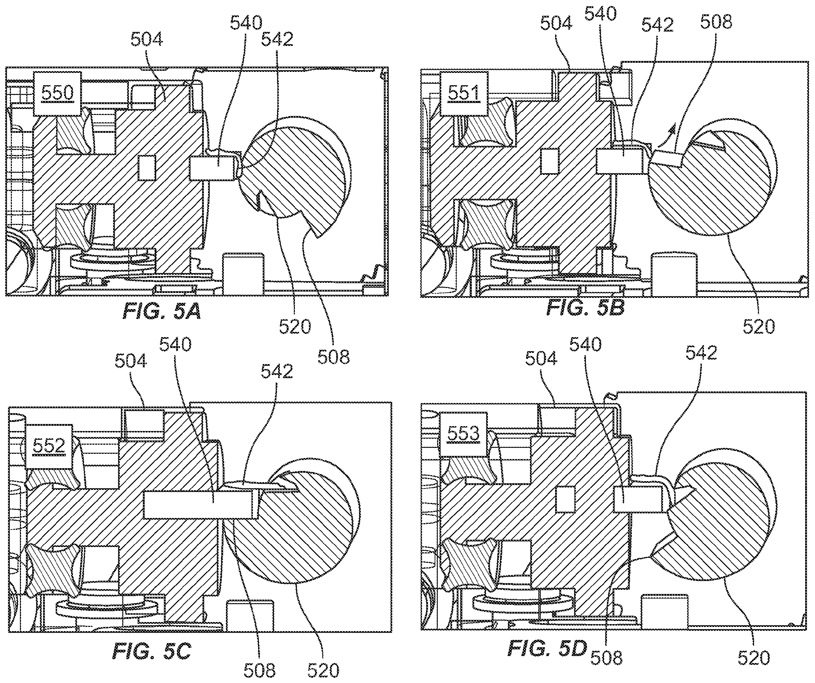

**FIGs. 5A-5D** illustrate an embodiment of a fluid delivery pump in accordance with the present disclosure. As shown in FIGs. 5A-5D, a fluid pump may include a piston 504 configured to engage a snail cam 520 having a drop off 508. In some embodiments, a flex sensor 542 may be used to detect the position of a rotating member, such as snail cam 520. Non-limiting examples of flex sensors may include a strain gauge, a piezoelectric element, a pressure sensor, and/or the like. For example, flex sensor 542 may be configured to detect drop off 508 of snail cam 520. In various embodiments, flex sensor 542 may be affixed to a portion of piston 504, such as a piston contact element 540. For example, flex sensor 542 may be wrapped around a portion of contact element, for example, such that when piston contact element 540 is approaching drop off 508 (for instance, the end of the snail cam ramp), flex sensor 542 may release, thereby generating a signal notifying that the pump has reached drop off 508. In some embodiments, flex sensor 542 may be wrapped around at least half of the extended distance of piston contact element 540.

As shown in step 550, piston 504 is engaging an outer surface of snail cam 520 at piston contact element 540. Flex sensor 542 is wrapped around an end of piston contact element 540 such that flex sensor 542 engages the outer surface of snail cam 520 and is in a flexed position (for instance, flexed toward piston 504). In some embodiments, flex sensor 542 may generate a signal indicating its current state (for instance, engaged with the outer surface of snail cam 520). At step 551, piston contact element 540 may be near drop off 508, for instance, near an edge of the snail cam ramp. Accordingly, flex sensor 542 may partially release and send a signal indicating its current state (for instance, in a partial-release form indicating piston 504 is nearing drop off 508).

At step 552, piston contact element 540 may have entered drop off 508 and flex sensor 542 may be fully or substantially fully extended. Accordingly, flex sensor 542 may generate a signal indicating its current state (for instance, in a fully-release form indicating piston 504 is within drop off 508). At step 553, piston contact element 540 may be exiting drop off 508 and re-engaging the outer surface or ramp of snail cam 520. Accordingly, flex sensor 542 may reflex and generate a signal indicating its current state (for instance, in a fully-flexed form indicating piston 504 is engaging the ramp of snail cam 520).

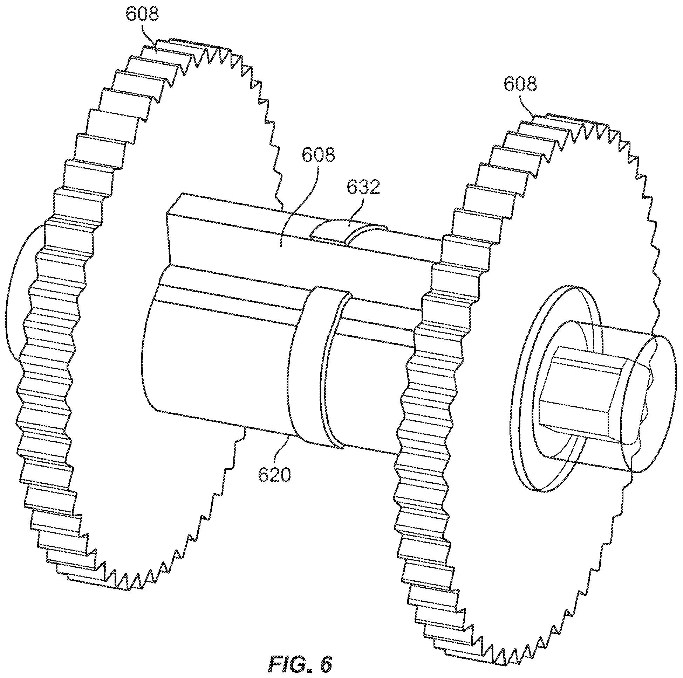

**FIG. 6** illustrates an embodiment of a rotary element of a fluid delivery pump in accordance with the present disclosure. As shown in FIG. 6, an electrical element 632 may be applied along the circumference of a snail cam 608 that may provide for a continuous positional sensor. In some embodiments, a corresponding electrical element (for instance, the same or similar to electrical element 440 on piston 404 of FIGs. 4A-4B) may ride along electrical element 632 to form a circuit when a pump element, such as a piston, engages an outer surface or ramp of snail cam 620. In general, the embodiment depicted in FIG. 6 provides an alternative arrangement to the example depicted in FIGs. 4A-4B. For example, in FIGs. 4A-4B a circuit 434 is formed responsive to piston 404 engaging or being within a specified threshold of drop off 408; otherwise, no circuit is formed. In the embodiment of FIG. 6, a circuit may be formed when a piston (not shown) rides along electrical element 632 over the ramp of snail cam 620; a circuit is not formed when the piston is at or within a specified threshold of drop off 608.

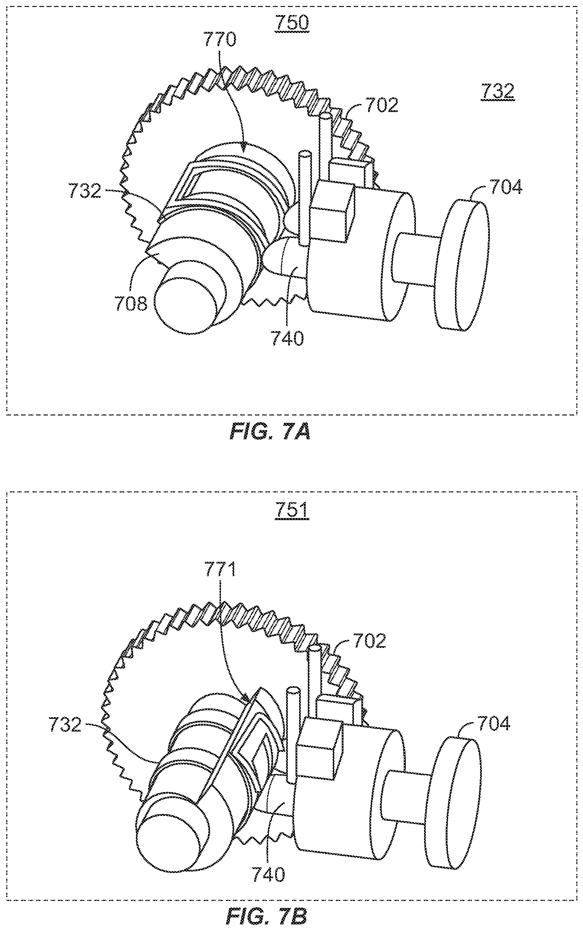

In the embodiment shown in FIG. 6, a piston shaft (or other pump component) may be in contact with a conductive strip to complete a circuit (in some embodiments, circuit may be a resistive circuit). As the ratchet 608 spins snail cam 620, the circuit grows along with the resistance which can then be measured at any time to get the position (see also, FIGs. 7A-7B). For example, as snail cam 620 rotates, the formed circuit becomes shorter until dropping over or just before dropping over the edge of drop off 608. As the circuit shortens, resistance may change (e.g., decrease), allowing monitoring the position of snail cam 620 and/or piston. It may be easily determined beforehand what resistance values correspond to what particular angular orientations of the snail cam 620. Accordingly, knowing the measured resistance value will indicate what angular position snail cam 620 is in, and hence, may indicate when to switch from a fluid dispensing path to a fluid filling path.

**FIGs. 7A-7B** illustrate an embodiment of a fluid delivery pump in accordance with the present disclosure. As shown in FIGs. 7A-7B, a fluid pump may include a ratchet 702 operably coupled to a snail cam 720 having a drop off 708. An electrical element 732 (such as a resistive strip) may be arranged along a ramp or surface of snail cam 720 (and not on the vertical drop off surface). A corresponding electrical element 740 may be arranged on a piston 704 that is operative to engage snail cam 720 as ratchet 702 rotates. Engagement of electrical elements 732 and 740 may complete a circuit. FIGs. 7A-7B depicts a first state 750 with a long path length 770 (and a first resistance value) and a second state 750 with a shorter path length 771 (and a second resistance value less than the first resistance value). Snaking resistive strip 732, for example, to double or otherwise increase the length of a circuit formed via resistive strip 732 and a corresponding electrical element 740 (for instance, as compared with the circuit formed from electrical element 632 in Fig. 6) may allow the change in resistance to double (or otherwise increase) as ratchet 702 and snail cam 720 rotate. The resistance change may be small, so the added resistance from doubling or increasing the length of the change in path may increase detectable resistance information.

It should be understood that while the use of words such as preferable, preferably, preferred or more preferred utilized in the description above indicate that the feature so described may be more desirable, it nonetheless may not be necessary and embodiments lacking the same may be contemplated as within the scope of the present disclosure, the scope being defined by the claims that follow. In reading the claims, it is intended that when words such as "a," "an," "at least one," or "at least one portion" are used there is no intention to limit the claim to only one item unless specifically stated to the contrary in the claim. When the language "at least a portion" and/or "a portion" is used the item can include a portion and/or the entire item unless specifically stated to the contrary.

## Claims

1. A fluid pump system for a wearable fluid delivery device (205), comprising:
a control system;
a first pump element comprising a piston (404) and associated with a first electrical element (440); and
a second pump element comprising a snail cam (420) having a ramp and a drop off (408) at an end of the ramp and associated with a second electrical element (432);
wherein the first pump element is configured to engage the second pump element to form an electrical circuit between the first electrical element and the second electrical element, the control system configured to receive at least one signal from the electrical circuit, wherein the second electrical element (432) is arranged at a position at or near the drop off (408) such that a closed circuit is formed and causes the at least one signal to be sent to indicate when the piston (404) is about to reach or has reached the drop off (408).

2. The fluid pump system of claim 1, the first electrical element and the second electrical element comprising at least one conductive material.

3. The fluid pump system of claim 1, the wearable fluid delivery device comprising an insulin infusion device.

4. The fluid pump system of claim 1, the first electrical element comprising at least one projection extending from a portion of the piston engaging the ramp of the snail cam.

5. The fluid pump system of claim 4, the second electrical element comprising at least one conductive strip, the conductive strip arranged to form the circuit to indicate when the piston has reached the drop off.

6. The fluid pump system of claim 5, the second electrical element arranged around a circumference of the snail cam except for the drop off.

7. The fluid pump system of claim 6, the second electrical element arranged to open the circuit to indicate when the piston has reached the drop off.

8. The fluid pump system of claim 1, the first electrical element to engage the second electrical element to form a circuit as the piston engages the snail cam ramp during rotation of the snail cam.

9. The fluid pump system of claim 1, the signal comprising a resistance value configured to indicate a position of the piston on the ramp of the snail cam.

10. The fluid pump system of claim 1, the control system to determine pump information based on the signal.

11. The fluid pump system of claim 10, the control system to change a fluid path of the fluid pump system based on the signal.

12. The fluid pump system of claim 1, wherein
the first pump element is configured to rotate; and
the second electrical element is a flexible electrical element configured to engage the first pump element;
wherein the flexible electrical element is configured to have a plurality of flex states based on a type of engagement with the first pump element,
wherein the control system is configured to receive at least one signal from the flexible electrical element indicating at least one of the plurality of flex states.

13. The fluid pump system of claim 12, the flexible electrical element comprising at least one of a strain gauge, a piezoelectrical element, or a pressure sensor.

14. The fluid pump system of claim 1, further comprising:
a reservoir to store a fluid; and
a needle to infuse the fluid into a patient.

15. The fluid pump system of claim 14, wherein the fluid is insulin.

## Patentansprüche

1. Fluidpumpensystem für eine tragbare Fluidfördervorrichtung (205), das Folgendes umfasst:
ein Steuersystem;
ein erstes Pumpenelement, das einen Kolben (404) umfasst und mit einem ersten elektrischen Element (440) assoziiert ist; und
ein zweites Pumpenelement, das einen Schneckennocken (420) mit einer Rampe und einem Abfall (408) an einem Ende der Rampe umfasst und mit einem zweiten elektrischen Element (432) assoziiert ist;
wobei das erste Pumpenelement dazu ausgelegt ist, mit dem zweiten Pumpenelement in Eingriff zu kommen, um einen Stromkreis zwischen dem ersten elektrischen Element und dem zweiten elektrischen Element zu bilden, wobei das Steuersystem dazu ausgelegt ist, mindestens ein Signal von dem Stromkreis zu empfangen, wobei das zweite elektrische Element (432) an einer Position an oder in der Nähe des Abfalls (408) angeordnet ist, sodass ein geschlossener Kreis gebildet wird und bewirkt, dass das mindestens eine Signal gesendet wird, um anzuzeigen, wann der Kolben (404) dabei ist, den Abfall (408) zu erreichen, oder ihn erreicht hat.

2. Fluidpumpensystem nach Anspruch 1, wobei das erste elektrische Element und das zweite elektrische Element mindestens ein leitfähiges Material umfassen.

3. Fluidpumpensystem nach Anspruch 1, wobei die tragbare Fluidfördervorrichtung eine Insulininfusionsvorrichtung umfasst.

4. Fluidpumpensystem nach Anspruch 1, wobei das erste elektrische Element mindestens einen Vorsprung umfasst, der sich von einem Abschnitt des Kolbens erstreckt, der mit der Rampe des Schneckennockens in Eingriff steht.

5. Fluidpumpensystem nach Anspruch 4, wobei das zweite elektrische Element mindestens einen leitfähigen Streifen umfasst, wobei der leitfähige Streifen so angeordnet ist, dass er den Kreis bildet, um anzuzeigen, wann der Kolben den Abfall erreicht hat.

6. Fluidpumpensystem nach Anspruch 5, wobei das zweite elektrische Element um einen Umfang des Schneckennockens herum mit Ausnahme des Abfalls angeordnet ist.

7. Fluidpumpensystem nach Anspruch 6, wobei das zweite elektrische Element so angeordnet ist, dass es den Kreis öffnet, um anzuzeigen, wann der Kolben den Abfall erreicht hat.

8. Fluidpumpensystem nach Anspruch 1, wobei das erste elektrische Element mit dem zweiten elektrischen Element in Eingriff steht, um einen Kreis zu bilden, wenn der Kolben während der Drehung des Schneckennockens mit der Schneckennockenrampe in Eingriff steht.

9. Fluidpumpensystem nach Anspruch 1, wobei das Signal einen Widerstandswert umfasst, der dazu ausgelegt ist, eine Position des Kolbens auf der Rampe des Schneckennockens anzugeben.

10. Fluidpumpensystem nach Anspruch 1, wobei das Steuersystem dazu ausgelegt ist, Pumpeninformationen basierend auf dem Signal zu bestimmen.

11. Fluidpumpensystem nach Anspruch 10, wobei das Steuersystem dazu ausgelegt ist, einen Fluidpfad des Fluidpumpensystems basierend auf dem Signal zu ändern.

12. Fluidpumpensystem nach Anspruch 1, wobei:
das erste Pumpenelement dazu ausgelegt ist, sich zu drehen; und
das zweite elektrische Element ein flexibles elektrisches Element ist, das dazu ausgelegt ist, mit dem ersten Pumpenelement in Eingriff zu treten;
wobei das flexible elektrische Element dazu ausgelegt ist, mehrere Biegezustände basierend auf einer Art von Eingriff mit dem ersten Pumpenelement aufzuweisen,
wobei das Steuersystem dazu ausgelegt ist, mindestens ein Signal von dem flexiblen elektrischen Element zu empfangen, das mindestens einen der mehreren Biegezustände angibt.

13. Fluidpumpensystem nach Anspruch 12, wobei das flexible elektrische Element einen Dehnungsmessstreifen und/oder ein piezoelektrisches Element und/oder einen Drucksensor umfasst.

14. Fluidpumpensystem nach Anspruch 1, ferner umfassend:
ein Reservoir zum Speichern eines Fluids; und
eine Nadel zum Infundieren des Fluids in einen Patienten.

15. Fluidpumpensystem nach Anspruch 14, wobei das Fluid Insulin ist.

## Revendications

1. Système de pompe à fluide pour un dispositif de distribution de fluide pouvant être porté (205), comprenant :
un système de commande ;
un premier élément de pompe comprenant un piston (404) et associé à un premier élément électrique (440) ; et
un second élément de pompe comprenant une came escargot (420) ayant une rampe et une chute (408) au niveau d'une extrémité de la rampe et associé à un second élément électrique (432) ;
dans lequel le premier élément de pompe est configuré pour se mettre en prise avec le second élément de pompe afin de former un circuit électrique entre le premier élément électrique et le second élément électrique, le système de commande configuré pour recevoir au moins un signal en provenance du circuit électrique, dans lequel le second élément électrique (432) est agencé au niveau d'une position au niveau ou à proximité de la chute (408) de telle sorte qu'un circuit fermé soit formé et amène l'au moins un signal à être envoyé pour indiquer quand le piston (404) est sur le point d'atteindre ou a atteint la chute (408).

2. Système de pompe à fluide selon la revendication 1, le premier élément électrique et le second élément électrique comprenant au moins un matériau conducteur.

3. Système de pompe à fluide selon la revendication 1, le dispositif de distribution de fluide pouvant être porté comprenant un dispositif de perfusion d'insuline.

4. Système de pompe à fluide selon la revendication 1, le premier élément électrique comprenant au moins une saillie s'étendant à partir d'une partie du piston se mettant en prise avec la rampe de la came escargot.

5. Système de pompe à fluide selon la revendication 4, le second élément électrique comprenant au moins une bande conductrice, la bande conductrice agencée pour former le circuit afin d'indiquer quand le piston a atteint la chute.

6. Système de pompe à fluide selon la revendication 5, le second élément électrique étant agencé autour d'une circonférence de la came escargot à l'exception de la chute.

7. Système de pompe à fluide selon la revendication 6, le second élément électrique agencé pour ouvrir le circuit afin d'indiquer quand le piston a atteint la chute.

8. Système de pompe à fluide selon la revendication 1, dans lequel le premier élément électrique se met en prise avec le second élément électrique pour former un circuit à mesure que le piston se met en prise avec la rampe de came escargot pendant la rotation de la came escargot.

9. Système de pompe à fluide selon la revendication 1, le signal comprenant une valeur de résistance configurée pour indiquer une position du piston sur la rampe de la came escargot.

10. Système de pompe à fluide selon la revendication 1, le système de commande servant à déterminer des informations de pompe sur la base du signal.

11. Système de pompe à fluide selon la revendication 10, le système de commande étant destiné à changer un trajet de fluide du système de pompe à fluide sur la base du signal.

12. Système de pompe à fluide selon la revendication 1, dans lequel
le premier élément de pompe est configuré pour tourner ; et
le second élément électrique est un élément électrique flexible configuré pour se mettre en prise avec le premier élément de pompe ;
dans lequel l'élément électrique flexible est configuré pour avoir une pluralité d'états de flexion basés sur un type de mise en prise avec le premier élément de pompe,
dans lequel le système de commande est configuré pour recevoir au moins un signal en provenance de l'élément électrique flexible indiquant au moins un de la pluralité d'état de flexion.

13. Système de pompe à fluide selon la revendication 12, l'élément électrique flexible comprenant au moins l'un d'une jauge de contrainte, d'un élément piézoélectrique, ou d'un capteur de pression.

14. Système de pompe à fluide selon la revendication 1, comprenant en outre :
un réservoir pour stocker un fluide ; et
une aiguille pour perfuser le fluide à un patient.

15. Système de pompe à fluide selon la revendication 14, dans lequel le fluide est de l'insuline.
